# EUROPEAN PATENT SPECIFICATION

(11) **EP 3 908 333 B1**
(45) Date of publication and mention of the grant of the patent: **13.05.2026**
(21) Application number: 20701128.9
(22) Date of filing: 06.01.2020
(51) Int. Cl.: A61L 2/10, G02B 6/00

(54) **DEVICE FOR DISINFECTING AT LEAST ONE OBJECT**
VORRICHTUNG ZUM DESINFIZIEREN MINDESTENS EINES GEGENSTANDES
DISPOSITIF POUR DÉSINFECTER AU MOINS UN OBJET

(30) Priority: 10.01.2019 NL 2022364
(43) Date of publication of application: 17.11.2021
(73) Proprietor: UV Smart Technologies B.V., 2289 EX Rijswijk (NL)
(72) Inventor: KEA, Thijs Joop, 2289 EX RIJSWIJK (NL); HOEK, Daan Jelmer, 2289 EX RIJSWIJK (NL)
(74) Representative: Algemeen Octrooi- en Merkenbureau B.V.
(86) International application number: PCT/NL2020/050003
(87) International publication number: WO 2020/145816

(56) References cited:
- EP-A1- 3 085 391
- WO-A1-2015/168111
- GB-A- 2 498 541
- US-B1- 7 138 087
- US-B1- 7 138 087
- US-B2- 6 919 057
- US-B2- 6 919 057
- US-B2- 8 636 950

## Description

The invention relates to a device for disinfecting at least one object, which device is provided with at least one UV-C light source by means of which UV-C light can be emitted in a closable chamber of the device.

The device described in US 2004/0099812 is able to disinfect objects in a closed chamber by means of UV-C light. With this known device, an object is placed in a receptacle by a user. The receptacle is configured as a kind of tray. However, the tray is not particularly suitable for all objects which can be disinfected by means of the known device, in particular for objects whose parts, if placed on the tray, may overlap. The surfaces of the overlapping parts are outside the reach of the UV-C light, as a result of which these parts of the object will not receive the dose of UV-C light prescribed for the disinfection of the object, as a result of which the object cannot be disinfected completely.

US 20140341777 describes a device for disinfecting at least one object, which device is provided with at least one UV-C light source by means of which UV-C light can be emitted in a closable chamber of the device, wherein at least one object-suspension element permeable to UV-C light may be provided in the chamber by means of which the object can be suspended in the chamber.

WO 2015/168111 discloses a disinfecting chamber with a hook transparent for UVC light, wherein an object to be disinfected can be attached to the hook. The hook may include a UVC light emitting diode (LED) or similar UVC light source.

It is therefore an object of the invention to provide a device which is improved compared to the known devices. In addition, it is a further object to provide a device which is easy to use and/or by means of which objects which are relatively difficult to clean using UV-C light can be disinfected in a quick and improved way.

This object is achieved by the device for disinfecting at least one object having the characteristics as defined in Claim 1.

The device is provided with at least one UV-C light source by means of which UV-C light can be emitted in a closable chamber of the device, wherein at least one object-suspension element permeable to UV-C light is provided in the chamber, by means of which the object can be suspended in the chamber. At least one UV-C light source is incorporated in the object-suspension element or a light conductor is provided in the object-suspension element, by means of which light from the at least one UV-C light source can be conducted to a position where a dose of UV-C light is to be delivered, so that light from the suspension element itself shines onto a surface of the object where this is suspended.

By incorporating the at least one UV-C light source or a light conductor connected thereto in the object-suspension element, a device is provided by means of which objects suspended in the chamber can be disinfected better, namely completely or virtually completely, by means of UV-C light. This device is particularly suitable for elongate objects which do not have a fixed shape, that is to say objects of which the parts to be disinfected may overlap if these objects are placed or deposited on a support. By only using UV-C light to perform the disinfection, it is also possible to disinfect (medical) electronic apparatus by means of this device. Examples of medical electronic objects/apparatus having no fixed shape are endoscopes, blood pressure cuffs and infusion pumps. By suspending such relatively long objects in a chamber which is closable for the UV-C treatment, such objects can be disinfected with a reduced or even virtually impossible risk of overlapping parts of the object forming shadows. Obviously, it is also possible to suspend dimensionally stable objects and objects of reduced longitudinal dimensions in the device.

Disinfection by means of UV-C light offers a user-friendly method for disinfecting objects. Using the correct dose of this light, it is possible to disinfect to at least log-5 (99.999% extermination) within a period of 20 to 60 seconds. Disinfection of an object by means of UV-C light does not have any, or only minimal, negative effects on the object to be disinfected. Also, a disinfection can be performed at relatively low temperatures (15-40 degrees Celsius) and due to the fact that no liquids or chemicals are used, the UV-C light disinfection process is quick, relatively safe and reliable and, in particular, suitable for electronic objects.

In order to ensure that the parts by which the object is suspended from the object-suspension element are irradiated with UV-C light during a treatment, the object-suspension element is made so as to be permeable to UV-C light, i.e. transparent to UV-C light, so that the parts of the object by means of which the object is suspended are likewise disinfected. The object-suspension element is preferably configured in such a way that it is suitable for suspending a large number of different objects, for example objects of different dimensions. The object-suspension element is made of materials which allow UV-C light to pass through and thus make disinfection through the object-suspension element possible, as a result of which UV-C light is able to reach the surface of the object which is in contact with the object-suspension element due to being suspended. The object support may be made, for example, of materials such as quartz glass, "soft glass" or Teflon.

Suspending the objects also offers the significant advantage that the object can be suspended in the device in a consistent manner, in a relatively quick and user-friendly manner. In this way, a relatively large number of objects can be disinfected per unit time.

Due to the large number of certain medical electronic objects/apparatus in hospitals, it is possible to fit out a device in a dedicated manner, for example adapted to only disinfect endoscopes or a certain type of endoscopes. The dimensions of the chamber or disinfection chamber, the light sources and the object-suspension element can then be designed and configured so as to be tailored specifically to a certain object.

In one aspect, the at least one object-suspension element may be provided with a displacement mechanism for displacing and/or rotating the object. In this way, the object can be displaced in the optimum position for receiving the disinfecting UV-C light after having been suspended. It has been found to be advantageous to operate the displacement mechanism during a UV-C light treatment in order to ensure in a more reliable way that all surfaces of the object are disinfected to a sufficient degree.

In a further aspect, the at least one object-suspension element may be displaceable in the chamber of the device by means of a displacement unit. Displacing the suspension element itself in the chamber may contribute further to the optimum disinfection-treatment of the object by the UV-C light. Displacing the displacement unit may take place before the object is suspended and/or before the treatment is started, in order to ensure that an object which is suspended therefrom is disinfectable by means of UV-C light while hanging freely. Hanging freely means that the object does not have further contact with the walls defining the chamber or with other components of the device, except for the object-suspension element.

The chamber may inter alia be defined by at least one UV-C light-reflecting wall. By means of at least one reflecting wall, a device can be made more compact and/or the UV-C light can be directed towards the object to be disinfected in a more concentrated way. The reflecting wall(s) or wall sections may comprise different finishes or coatings which are adapted, for example, to the specific application in order to achieve an optimum disinfection result on the objects. The finish may, for example, be matt or smooth or comprise a matt or smooth coating. Matt produces a diffuse distribution across a larger surface, while smooth is more suitable for directing and concentrating the light. The reflecting walls may largely define the disinfection chamber. The reflecting walls and/or wall sections may, for example, be made of Spectralon, PTFE, ePTFE, aluminium, magnesium oxide and zirconium oxide. The surface finish depends on the situation; varying from smooth glossy for concentrated light direction to relief for diffuse distribution.

Near a further UV-C light source, at least one displaceable UV-C light reflecting wall may be arranged in order to concentrate the UV-C light emitted by means of this UV-C light source onto the object to be disinfected.

The device has a relatively large height dimension compared to the width dimension and the length dimension. As a result thereof, it is possible to provide a chamber in the device which has a height dimension which is at least two times, preferably at least three times, larger than the largest of the two other dimensions, comprising the width dimension and the length dimension. In this way, elongate objects with a relatively large longitudinal dimension can be suspended in the chamber for a UV-C light treatment.

In one embodiment, the device comprises different light sources which extend in a vertical direction and which are arranged around the object-suspension element. Such an arrangement appears to disinfect objects particularly effectively.

The further light source may be provided with a displacement device for displacing the light source with respect to or in the chamber. In this way, UV-C light can be directed towards the object to be disinfected in a more concentrated way.

By means of image recognition via sensors coupled with a control unit of the device by means of which the displacement mechanism, the displacement unit and/or the displacement device can be controlled, the object can be given the optimum UV-C treatment.

In an alternative device which is optimized with regard to energy consumption, different preferably elongate light sources are provided which extend in a horizontal direction, and preferably extend parallel to each other, which are arranged above one another, wherein, viewed in a vertical direction of the chamber, a portion of the light sources can be switched off by means of a control unit of the device, depending on the dimensions of the object to be disinfected. In this way, it is possible, in the case of a suspended object which extends vertically, that is to say in the height direction, to a relatively limited extent, to switch off some of the light sources which the object is not directly in front of or near to in order to save energy. The device may be provided with sensors (for example cameras) connected to the control unit or the length of the object or the type of object may be input (by the user, via RFID, by means of a scanner) via an interface into the control unit of the device. By means of image recognition via the sensors or via the input by means of the interface coupled with the control unit of the device, the displacement mechanism, the displacement unit and/or the displacement device can be controlled, so that the object can be given an optimum UV-C treatment for the respective object. By means of image recognition via the sensors, a surface of an object to be cleaned can also be inspected before, after and/or during the UV-C treatment. By means of the contamination detected by means of image recognition via the sensors in combination with the control unit, the UV treatment can be modified before and/or during the UV-C treatment and/or be performed again/repeated. With a stationary object-suspension element, and in the case of a relatively short/small object, the bottom section of the light sources with respect to a section of the light sources which is situated further upwards can be switched off by means of the control unit in combination with the sensors or the interface, so that no unnecessary energy consumption takes place by light sources which do not, or only to a minimal degree, contribute to the disinfection treatment of this object.

By opting to suspend the apparatus in the case of disinfection of endoscope-like and echographic head-like apparatus, the length of the apparatus is less of an impediment to the disinfection process to be carried out using UV-C light.

It is also possible to iilluminate the object-suspension element by means of UV-C light. For example, it is possible to incorporate a light source in the object-suspension element, LEDs emitting UV-C light are particularly suitable for this purpose. For example, use may be made of light conductors in an object-suspension element which conduct light from a light source to a position where a UV-C light dose is to be delivered, so that light from the suspension element itself shines onto a surface of the object where this is suspended.

The above-described aspects will be explained below by means of exemplary embodiments in combination with the figures. However, the invention is not limited to the exemplary embodiment described below. Rather, a number of variants and modifications are possible which also use the inventive ideas and are therefore covered by the scope of protection. In particular, the possibility is mentioned of combining the characteristics/aspects which are only mentioned in the description and/or are shown in the figures with the characteristics of the claims insofar as they are compatible.
Fig. 1 shows a perspective view of a device for disinfecting an object, in particular an endoscope;
Fig. 2 shows a detail view of an object-suspension element of a device to which an object is attached;
Fig. 3 shows an alternative arrangement of light sources with reflecting walls, which arrangement can be used in a device for disinfecting an object;
Figs. 4a-e show a further variant of the device and details of this device;
Figs. 5a, b show yet another variant of the device and details of this device;
Figs. 6a, b show further details of components which may be used in a device for disinfecting an object, in particular an endoscope.

In the figures, identical components are denoted by the same reference numerals.

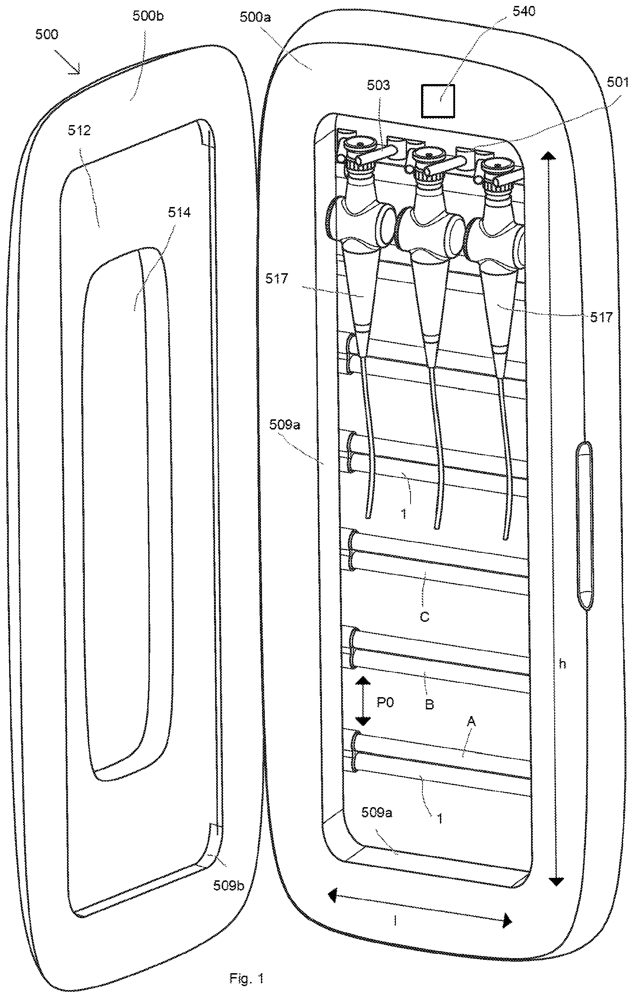

Fig. 1 shows a device 500 for disinfecting objects 517. This device 500 is provided with a number of object-suspension mechanisms 501, wherein an object, more particularly an endoscope 517, is suspended from each object-suspension mechanism 501. Of course, it is also possible to design the device 500 with only a single object-suspension mechanism.

The device 500 is provided with UV-C light sources 1, by means of which UV-C light can be emitted in a closable chamber of the device 500.

The device 500 comprises different elongate light sources 1 which extend in a horizontal direction, and preferably parallel to each other, which are arranged above one another. Viewed in a vertical direction, a portion of the light sources 1 can be switched off by means of a control unit, which is shown diagrammatically by square 540, depending of the dimensions of the object to be disinfected, so that no unnecessary energy consumption takes place by light sources 1 which do not, or only to a minimal degree, contribute to the disinfection treatment of the suspended object 517. In the example illustrated in Fig. 1, the bottom two or three UV-C lamp pairs A, B, C could be switched off for disinfecting the endoscopes 517.

The maximum operating temperature in the chamber of the device 500 is at most 50 degrees Celsius and no liquids are used for the disinfection, so that the device is suitable for disinfecting electronic objects.

The device 500 is provided with a stationary part 500a and with a part 500b which is hingeable about a hinge pin via hinges (not shown) and is movable between an open position in which the chamber is accessible and a closed position in which the chamber is closed. Both parts 500a, 500b may be provided with their own set of light sources 1, but in the illustrated example, only the stationary part 500a is provided with light sources 1. For safety reasons, disinfection is only to be performed in the closed position, that is to say in a closed chamber. The chamber is defined by walls, that is to say a glass panel (not shown in the diagrammatic view of Fig. 1) for covering the light sources 1, reflecting side walls 509a, 509b and a partly reflecting front wall 512 which is provided with a window 514 which does not allow any light to pass through which could be dangerous for a user when the device 500 is in use.

Above the light sources 1, the object-suspension mechanisms 501 are attached in the device. These object-suspension mechanisms 501 may be configured to be completely or partly permeable to UV-C light. Each object-suspension mechanism 501 comprises an object-suspension element 503 which is permeable to UV-C light and which, in the illustrated example, is formed by two bars between which a part of the object can be clamped, so that the object 517 is suspended by means of object-suspension element 503 of the object-suspension mechanism 501. The embodiment of the bars of the object-suspension mechanism 501 ensures that objects of variable dimensions can be suspended. The suspension operation which is intuitive for a user ensures that no additional training or learning is required by a user and that objects can be suspended in the device 500 quickly and in a consistent way. The object-suspension element 503 may be designed to have a displacement mechanism for displacing and/or rotating the object, for example during a UV-C light treatment. It is also possible to make the object-suspension mechanism 501 displaceable in its entirety by means of a displacement unit (not shown), for example via a rail guide (not shown), as a result of which the object-suspension mechanism 501 can be moved to a different height in the chamber automatically or by a user. Being able to move the object in the chamber is advantageous because the object can be positioned in the chamber so as to hang freely to perform a treatment by means of UV-C light under optimum conditions. Hang freely means that the object does not make contact with the walls defining the chamber or with any other components of the device, except for the contact with the object-suspension element 503.

The chamber comprises a height dimension h which is a number of times larger than the length dimension I of the chamber. The width dimension of the chamber illustrated in Fig. 1 is smaller than the length dimension I.

A number of or each light source(s) 1 (in Fig. 1 shown in pairs A-C) may be provided with a displacement device (not shown) for displacing the light source pairs in the directions indicated by arrow P0 with respect to the chamber in which the object is suspended.

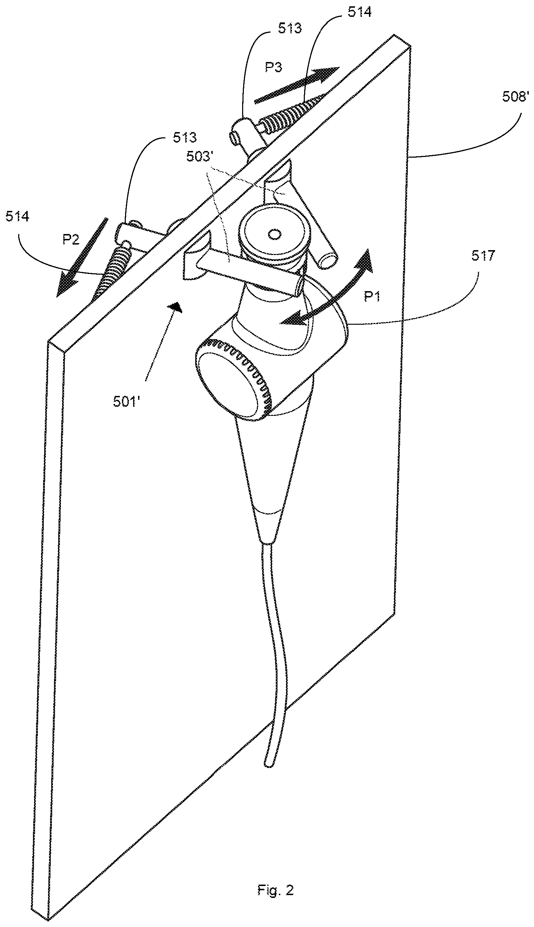

Fig. 2 shows a variant in which object-suspension mechanism 501' is fixed in the glass panel 508' by means of which the light sources 1 are covered. The suspension mechanism 501 itself is not displaceable with respect to the glass panel 508', but the bars 513 forming the object-suspension element 503' can be displaced by a user counter to the spring force (see arrows P2 and P3) in the directions indicated by arrow P1 in order to securely fix the object 517 between them.

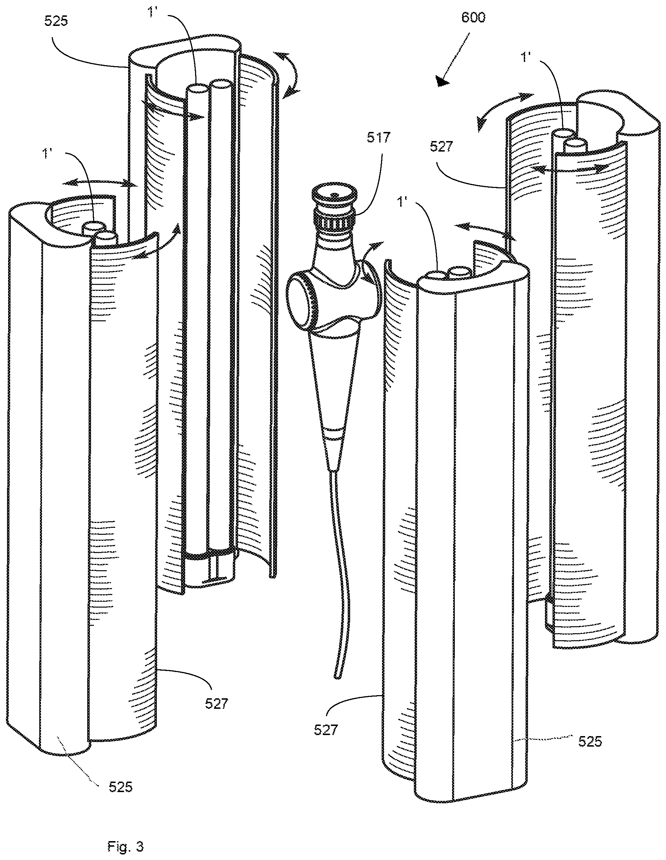

Fig. 3 shows a light source arrangement 600 which comprises different light sources 1' which extend in a vertical direction and which are arranged around the object-suspension element (not shown) and the object 517 suspended therefrom. The object-suspension element is provided with a displacement mechanism which can be operated during a UV-C light treatment in order to, for example, rotate the object 517 so that all surfaces of the object can be disinfected more effectively. Each of the light sources 1' of the arrangement 600 is surrounded by a reflector device 525 which is provided with displaceable UV-C light reflecting walls 527, which walls 527 are configured to be displaceable in order to concentrate the UV-C light emitted by means of the UV-C light source 1' more effectively onto the object to be disinfected 517. The walls 527 are displaceable in the directions indicated by the arrows in Fig. 3.

The light sources 1' and the reflecting walls 527 cooperating therewith preferably extend along virtually the entire height h of the chamber.

At least one UV-C light source (not shown) is incorporated in the object-suspension element 503; 503' or a light conductor (not shown) is provided in the bar-shaped object-suspension element, by means of which light from a UV-C light source (not shown) can be conducted to a position where a UV-C light dose is to be delivered, so that light from the suspension element 503; 503' itself shines onto a surface of the object 517 where this is suspended. When using light conductors, the UV-C light source (not shown) may, for example, be arranged behind the glass panel 508' illustrated in Fig. 2.

The object 517 illustrated in the Figs. 1-3 can be fitted in the device 500 by means of an object-suspension mechanism 501 / an object-suspension element, but, due to their design, certain objects can also be fitted in the device 500 using more than a single object-suspension mechanism 501 / a single object-suspension element 503, so that the surfaces of this object can be disinfected in the device 500 in an optimum manner.

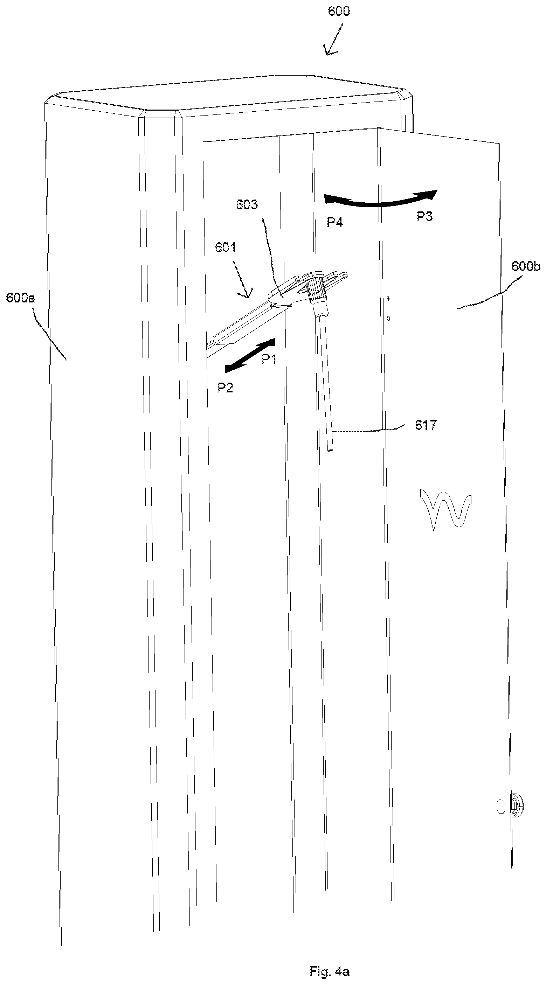

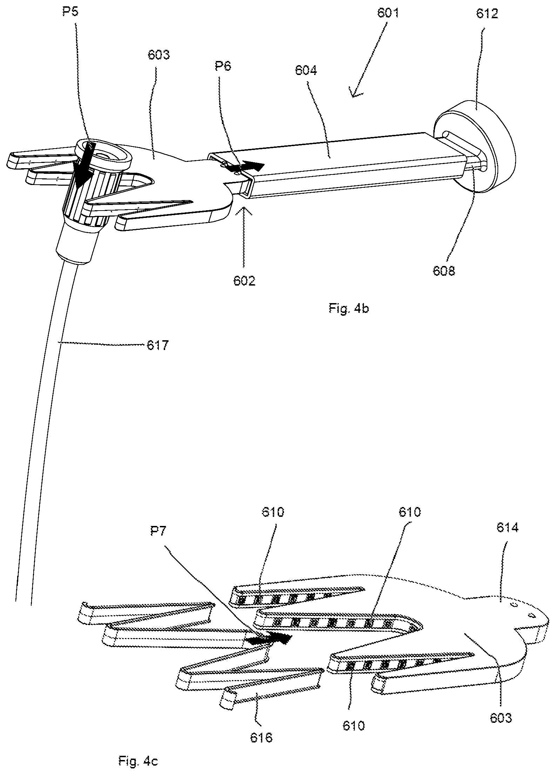

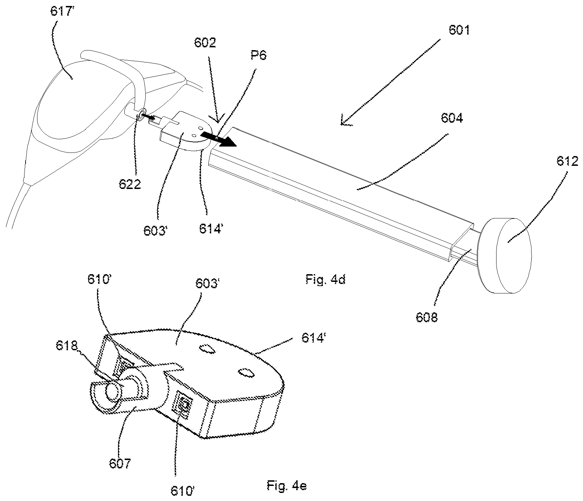

Figs. 4a-e show a further variant of the device 600 and detail views of components of this device 600. The cabinet-like device 600 is provided with a stationary part 600a and an access part 600b which is displaceable between an open position (as illustrated in Fig. 4a) in which the chamber is accessible and a closed position in which the chamber is closed. In the chamber, UV-C light sources (not shown) are arranged, for example in at least one wall of the stationary part 600a, for example corresponding to the light source arrangement illustrated in Fig. 1 or in the corners of the stationary part 600a, for example corresponding to a light source arrangement illustrated in Fig. 3. The device 600 is furthermore provided with an object-suspension mechanism 601 from which an object 617 can be suspended by means of an object-suspension element 603. The object-suspension mechanism 601 is designed to be displaceable by means of a displacement unit (not shown), as is shown by the double arrow which denotes the directions of displacement P1 and P2. By displacing the access part 600b from the closed position (in the direction P3 of the illustrated double arrow) to the open position, the at least one object-suspension element 603, by means of the displacement unit, is automatically displaceable from a working position situated in the chamber to a position situated at least partly outside the chamber and vice versa, that is to say back to the working position if the chamber is closed (direction P4 of the illustrated double arrow). In this way, when a chamber is open, an object can be fitted to the device 600 more easily and with more available space for a user. Also, the controlled mechanical movement by means of the displacement unit prevents the elongate object 617 from moving in an uncontrolled manner during the positioning of the object 617 to the working position in the chamber. The chamber may be relatively limited for certain shapes of objects, for example relatively long objects, as a result of which fitting such objects in the chamber is not only cumbersome for a user, but is also accompanied by the risk of damage to the object 617 or parts of the device 600 if the object is fitted in the device by the user in a relatively uncontrolled way. Figs. 4b-e show details of the object-suspension mechanism 601 and of object-suspension elements 603; 603' which can be releasably connected thereto. UV-C light sources 610; 610' are incorporated in the object-suspension elements 603; 603'. In the fork-like object-suspension element 603 (Fig. 4a-c), the UV-C light sources 610 are protected by means of a strip 616 which is permeable to UV-C light and which is attached to the object-suspension element 603 in the direction illustrated by arrow P7. Objects 517 can be fitted in the V-shaped fork recesses, with Fig. 4b showing by means of arrow P5 how an object 517 can be fitted/clamped into one of the fork recesses. In the illustrated example of the fork-like object-suspension element 603, V-shaped recesses of different dimensions are provided, so that objects 517 of different dimensions can be suspended in the device 600 by means of the object-suspension element 603. Of course, it is possible to provide the object-suspension element 603 with more or fewer recesses or with identically shaped V-shaped recesses. The device 600 is provided with differently designed object-suspension elements 603; 603' for carrying or attaching different shapes of objects or different types of objects, with the object-suspension mechanism 601 connected to the device 600 in each case being releasably connectable to one of the various object-suspension elements 603; 603'. To this end, the object-suspension mechanism 601 comprises a coupling part 604, one end of which is designed as a component of a quick coupling or click-fit mechanism 602 in order to bring about the releasable connection to an end 614, 614' of the various object-suspension elements 603; 603', which ends 614, 614' are also designed as a component of the quick coupling or the click-fit mechanism. Fitting object-suspension elements 603 to the coupling part is visualized in Fig. 4b by arrow P6. Furthermore, the coupling part 604 is displaceable with respect to support part 608 by means of the above-described displacement unit (see directions P1 and P2 in Fig. 4a). The support part 608 may be fixedly or hingeably displaceably attached to a support 612 connected to the device 600. If the support part 608 is hingeably displaceable, the object-suspension mechanism 601 comprises a further displacement unit (not shown). The object-suspension element 603' is configured to perform a pressure test on the object, in this case an endoscope 617'. Regulations may require certain medical objects, such as for example endoscopes, to be tested for leaks by means of a pressure tester (not shown). Such a pressure tester may be incorporated in the device 600 or may be used in combination with the device 600, wherein such a pressure tester is used to check if no cracks or leaks have formed in the object where accumulation of dirt and the associated risk of infection may ensue. A pressure tester may be provided/incorporated in the suspension element and the suspension mechanism, for example, in particular for endoscopes, wherein the pressure test can be performed by connecting a pressure line 618 from the object-suspension element 603' to a valve 622 of the endoscope 617' and by bringing and keeping the combination at a predetermined pressure for a certain period of time. By combining a pressure test with a UV-C light treatment in the device, time can be saved in preparing the endoscope for a subsequent use. It is even possible to perform the pressure test simultaneously with the UV-C light treatment in the device 600. By means of a leakage-test pressure nipple 607 of the object-suspension element 603', the endoscope 617' can be fitted to / suspended from the object-suspension element 603' and thus to/from the object-suspension mechanism 601 in the chamber of the device in order to perform a disinfection by means of UV-C light and/or a pressure test. The pressure-test pressure nipple 607 of the object-suspension element 603' may also be provided with a UV-C light source (not shown) incorporated therein, so that the contact surfaces of the valve 622 and surrounding parts of the endoscope 617' can also be treated with UV-C light, so that a maximum disinfection of the endoscope 617' can be ensured.

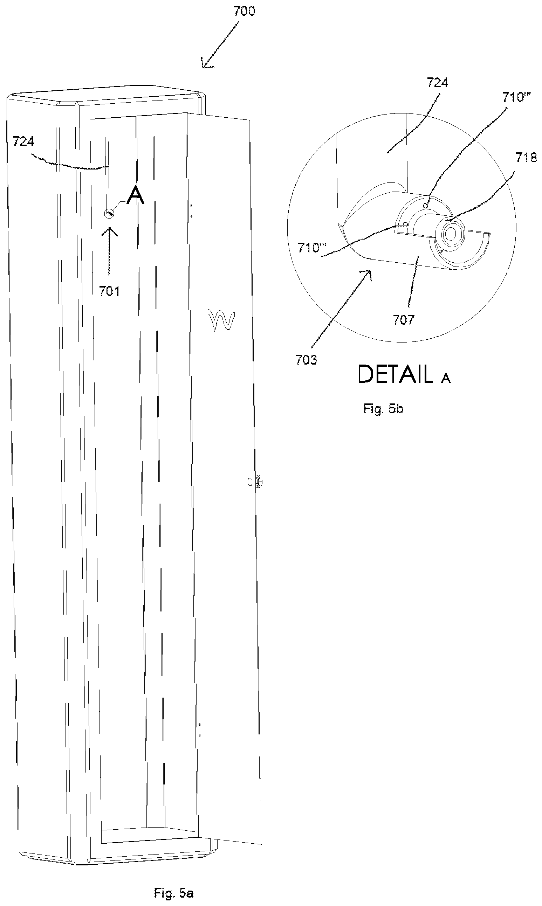

Fig. 5a illustrates a device 700 which is virtually identical to the one illustrated in Fig. 4a, wherein the device 700 only differs in that the displaceable suspension mechanism 701 is designed differently. The object-suspension element 703, which is shown in detail in Fig. 5b, is configured (just like the object-suspension element 603') to perform a pressure test on an endoscope (not shown) and is displaceable via a displacement member 724 of the suspension mechanism 701, for example in a way similar to that described above for the suspension mechanism 601. The displacement member 724 comprises a pressure line which is connected to a pressure source in order to be able to perform a pressure test. The object-suspension element 703 is formed as a leakage-test pressure nipple 707 which is designed in a similar way to the above-described leakage-test pressure nipple 607 and is thus provided with corresponding reference numerals incremented by one hundred in order to denote the corresponding components thereof. In the object-suspension element 703, the UV-C light sources 710"' incorporated therein are actually visible.

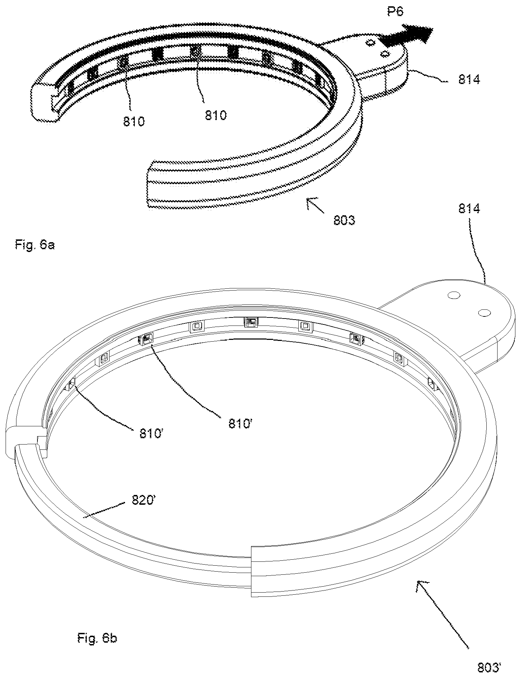

Figs. 6a and 6b show alternative annular object-suspension elements 803; 803' which can be connected to the suspension mechanism 601 illustrated in the Figs. 4a, 4b by means of an end 814; 814'. UV-C light sources 810; 810' are incorporated in the object-suspension elements 803; 803'. The object-suspension element 803' differs from the object-suspension element 803 in that the object-suspension element 803' is provided with a displaceable portion 820' by means of which a closed ring shape can be brought about, so that an object is fitted non-releasably, or at least not easily removably in the object-suspension element 803' by the user until a disinfection treatment with UV-C light and using the device has finished. In this way, the quality of the disinfection process to be performed can be guaranteed. A user can only open the ring shape of object-suspension element 803' by means of an additional security operation when a disinfection treatment on the object has not been performed completely, wherein the user is made aware of the risks and consequences of an object which has not undergone the prescribed disinfection treatment. Obviously, it is possible to design the other suspension mechanisms/suspension elements illustrated/discussed in this document in a similar manner. The release of the object may be controlled by means of the control unit and the image recognition device with the sensors, after these have established if the desired (predetermined) degree of cleaning of the object by UV-C light has been achieved, so that a certain quality requirement is met. Instead of the object-suspension element, a similar quality control may be achieved, if desired, by means of the closable chamber, if the latter remains inaccessible until a disinfection treatment using UV-C light which is to be performed with the device has finished or until, again, an additional security operation has been performed by a user.

The annular object-suspension elements 803; 803' illustrated in the Figs. 6a and 6b may also have the function of a guide element for guiding elongate objects in the chamber at a vertical distance from the suspension element. To this end, the respective device (not shown) then does have to be provided with at least two object-suspension mechanisms which are arranged at a vertical distance in the chamber (for example two object-suspension mechanisms which are shown in Fig. 4a). Furthermore, the guide elements may be designed in an identical manner to the object-suspension elements 803; 803' which is, however, not necessary or desirable with regard to dimensions, if for example the shape of the object to be suspended does not stay the same in the length direction.

## Claims

1. Device (600; 700) for disinfecting at least one object (517; 617; 617'), which device is provided with at least one UV-C light source (610'; 710') by means of which UV-C light can be emitted in a closable chamber of the device, wherein at least one object-suspension element (603'; 703) permeable to UV-C light is provided in the chamber, by means of which the object can be suspended in the chamber, wherein the at least one UV-C light source (610'; 710') is incorporated in the object-suspension element, or
in that a light conductor is provided in the object-suspension element, by means of which light from the at least one UV-C light source can be conducted to a position where a dose of UV-C light is to be delivered, so that light from the suspension element itself shines onto a surface of the object where this is suspended, **characterized**
**in that** the object-suspension element (603'; 703) is configured to perform a pressure test on the object.

2. Device (600; 700) according to Claim 1, wherein the at least one object-suspension element is provided with a displacement mechanism for displacing and/or rotating the object.

3. Device (600; 700) according to Claim 2, wherein the displacement mechanism is operable during a UV-C light treatment.

4. Device (600) according to one of the preceding claims, wherein the at least one object-suspension element is displaceable (P1, P2) in the chamber of the device by means of a displacement unit.

5. Device (600; 700) according to Claim 4, wherein the at least one object-suspension element is displaceable so that an object which is suspended therefrom is disinfectable by means of UV-C light while hanging freely.

6. Device (600; 700) according to one of the preceding claims, wherein the device is provided with at least one guide element (803; 803') for guiding elongate objects in the chamber at a vertical distance from the suspension element.

7. Device (600; 700) according to Claim 6, wherein a further UV-C light source (810; 810') is incorporated in the at least one guide element, or
wherein a light conductor is provided in the guide element by means of which light from a further UV-C light source can be conducted to a position where a dose of UV-C light has to be delivered, so that light from the guide element itself shines onto a surface of the object which faces the guide element.

8. Device (600; 700) according to one of the preceding claims, wherein a further UV-C light source (1; 1') is provided with a displacement device for displacing the light source with respect to or in the chamber.

9. Device (600; 700) according to Claim 1, wherein a pressure test can be performed by means of the object-suspension element by connecting a pressure line (618) in the object-suspension element to a valve (522) of a medical object, such as for example an endoscope.

10. Device (600; 700) according to Claim 1 or 9, wherein an object can be fitted to the object-suspension element by means of a leakage-test pressure nipple (607; 707) of the object-suspension element (603'; 703).

11. Device (600) according to one of the preceding claims, wherein the device (600) is provided with differently designed suspension elements (603; 603') for carrying or attaching different shapes of objects or different types of objects, and with at least one object-suspension mechanism (601) by means of which in each case one of the various object-suspension elements can be releasably connected.

12. Device (600) according to Claim 11, wherein the object-suspension mechanism (601) is configured for performing a pressure test on an object.

## Patentansprüche

1. Vorrichtung (600; 700) zum Desinfizieren mindestens eines Gegenstandes (517; 617; 617'), wobei die Vorrichtung mit mindestens einer UV-C-Lichtquelle (610'; 710') bereitgestellt ist, mittels der UV-C-Licht in einer verschließbaren Kammer der Vorrichtung emittiert werden kann, wobei mindestens ein Gegenstandsaufhängungselement (603'; 703), das für UV-C-Licht durchlässig ist, in der Kammer bereitgestellt ist, mittels dessen der Gegenstand in der Kammer aufgehängt werden kann, wobei die mindestens eine UV-C-Lichtquelle (610'; 710') in das Gegenstandsaufhängungselement integriert ist, oder
dass ein Lichtleiter in dem Gegenstandsaufhängungselement bereitgestellt ist, mittels dessen Licht von der mindestens einen UV-C-Lichtquelle zu einer Position geleitet werden kann, an der eine Dosis UV-C-Licht abzugeben ist, sodass Licht von dem Aufhängungselement selbst auf eine Oberfläche des Gegenstandes scheint, an der dieser aufgehängt ist, **dadurch gekennzeichnet,**
**dass** das Gegenstandsaufhängungselement (603'; 703) dazu konfiguriert ist, einen Drucktest an dem Gegenstand durchzuführen.

2. Vorrichtung (600; 700) nach Anspruch 1, wobei das mindestens eine Gegenstandsaufhängungselement mit einem Verschiebungsmechanismus zum Verschieben und/oder Drehen des Gegenstandes bereitgestellt ist.

3. Vorrichtung (600; 700) nach Anspruch 2, wobei der Verschiebungsmechanismus während einer UV-C-Lichtbehandlung bedienbar ist.

4. Vorrichtung (600) nach einem der vorhergehenden Ansprüche, wobei das mindestens eine Gegenstandsaufhängungselement in der Kammer der Vorrichtung mittels einer Verschiebungseinheit verschiebbar ist (P1, P2).

5. Vorrichtung (600; 700) nach Anspruch 4, wobei das mindestens eine Gegenstandsaufhängungselement verschiebbar ist, sodass ein Gegenstand, der daran aufgehängt ist, mittels UV-C-Licht desinfizierbar ist, während er frei hängt.

6. Vorrichtung (600; 700) nach einem der vorhergehenden Ansprüche, wobei die Vorrichtung mit mindestens einem Führungselement (803; 803') zum Führen von länglichen Gegenständen in der Kammer in einem vertikalen Abstand von dem Aufhängungselement bereitgestellt ist.

7. Vorrichtung (600; 700) nach Anspruch 6, wobei eine weitere UV-C-Lichtquelle (810; 810') in das mindestens eine Führungselement integriert ist, oder
wobei ein Lichtleiter in dem Führungselement bereitgestellt ist, mittels dessen Licht von einer weiteren UV-C-Lichtquelle zu einer Position geleitet werden kann, an der eine Dosis UV-C-Licht abzugeben ist, sodass Licht von dem Führungselement selbst auf eine Oberfläche des Gegenstandes scheint, die dem Führungselement zugewandt ist.

8. Vorrichtung (600; 700) nach einem der vorhergehenden Ansprüche, wobei eine weitere UV-C-Lichtquelle (1; 1') mit einer Verschiebungsvorrichtung zum Verschieben der Lichtquelle in Bezug auf oder in der Kammer bereitgestellt ist.

9. Vorrichtung (600; 700) nach Anspruch 1, wobei ein Drucktest mittels des Gegenstandsaufhängungselements durch Verbinden einer Druckleitung (618) in dem Gegenstandsaufhängungselement mit einem Ventil (522) eines medizinischen Gegenstandes, wie zum Beispiel eines Endoskops, durchgeführt werden kann.

10. Vorrichtung (600; 700) nach Anspruch 1 oder 9, wobei ein Gegenstand an dem Gegenstandsaufhängungselement mittels eines Lecktestdrucknippels (607; 707) des Gegenstandsaufhängungselements (603'; 703) angebracht werden kann.

11. Vorrichtung (600) nach einem der vorhergehenden Ansprüche, wobei die Vorrichtung (600) mit unterschiedlich gestalteten Aufhängungselementen (603; 603') zum Tragen oder Befestigen von verschiedenen Formen von Gegenständen oder verschiedenen Arten von Gegenständen und mit mindestens einem Gegenstandsaufhängungsmechanismus (601), mittels dessen in jedem Fall eines der verschiedenen Gegenstandsaufhängungselemente lösbar verbunden werden kann, bereitgestellt ist.

12. Vorrichtung (600) nach Anspruch 11, wobei der Gegenstandsaufhängungsmechanismus (601) dazu konfiguriert ist, einen Drucktest an einem Gegenstand durchzuführen.

## Revendications

1. Dispositif (600 ; 700) destiné à désinfecter au moins un objet (517 ; 617 ; 617'), lequel dispositif est pourvu d'au moins une source de lumière UV-C (610' ; 710') au moyen de laquelle une lumière UV-C peut être émise dans une chambre pouvant être fermée du dispositif, au moins un élément de suspension d'objet (603' ; 703) perméable à la lumière UV-C étant prévu dans la chambre, au moyen duquel l'objet peut être suspendu dans la chambre, ladite au moins une source de lumière UV-C (610' ; 710') étant incorporée dans l'élément de suspension d'objet, ou
en ce qu'un conducteur de lumière est prévu dans l'élément de suspension d'objet, au moyen duquel la lumière provenant de l'au moins une source de lumière UV-C peut être conduite jusqu'à une position où une dose de lumière UV-C doit être distribuée, afin que la lumière provenant de l'élément de suspension lui-même rayonne sur une surface de l'objet où celui-ci est suspendu, **caractérisé**
**en ce que** l'élément de suspension d'objet (603' ; 703) est conçu pour réaliser un essai de pression sur l'objet.

2. Dispositif (600 ; 700) selon la revendication 1, ledit au moins un élément de suspension d'objet étant pourvu d'un mécanisme de déplacement pour déplacer et/ou faire tourner l'objet.

3. Dispositif (600 ; 700) selon la revendication 2, ledit mécanisme de déplacement pouvant fonctionner durant un traitement par lumière UV-C.

4. Dispositif (600) selon l'une des revendications précédentes, ledit au moins un élément de suspension d'objet pouvant être déplacé (P1, P2) dans la chambre du dispositif au moyen d'une unité de déplacement.

5. Dispositif (600 ; 700) selon la revendication 4, ledit au moins un élément de suspension d'objet pouvant être déplacé afin qu'un objet qui est suspendu à celui-ci puisse être désinfecté au moyen d'une lumière UV-C tout en étant suspendu librement.

6. Dispositif (600 ; 700) selon l'une des revendications précédentes, ledit dispositif étant pourvu d'au moins un élément de guidage (803 ; 803') destiné à guider des objets allongés dans la chambre à une distance verticale à partir de l'élément de suspension.

7. Dispositif (600 ; 700) selon la revendication 6, une source de lumière UV-C supplémentaire (810 ; 810') étant incorporée dans ledit au moins un élément de guidage, ou
un conducteur de lumière étant prévu dans l'élément de guidage au moyen duquel la lumière provenant d'une source de lumière UV-C supplémentaire peut être conduite jusqu'à une position où une dose de lumière UV-C doit être distribuée, afin que la lumière provenant de l'élément de guidage lui-même rayonne sur une surface de l'objet qui fait face à l'élément de guidage.

8. Dispositif (600 ; 700) selon l'une des revendications précédentes, une source de lumière UV-C supplémentaire (1 ; 1') étant pourvue d'un dispositif de déplacement destiné à déplacer la source de lumière par rapport à la chambre ou dans celle-ci.

9. Dispositif (600 ; 700) selon la revendication 1, un essai de pression pouvant être réalisé au moyen de l'élément de suspension d'objet en raccordant une conduite de pression (618) dans l'élément de suspension d'objet à une soupape (522) d'un objet médical, tel que par exemple un endoscope.

10. Dispositif (600 ; 700) selon la revendication 1 ou 9, un objet pouvant être ajusté sur l'élément de suspension d'objet au moyen d'un mamelon de pression d'essai de fuite (607 ; 707) de l'élément de suspension d'objet (603' ; 703).

11. Dispositif (600) selon l'une des revendications précédentes, ledit dispositif (600) étant pourvu d'éléments de suspension de conception différente (603 ; 603') pour porter ou fixer différentes formes d'objets ou différents types d'objets, et d'au moins un mécanisme de suspension d'objet (601) au moyen duquel, dans chaque cas, l'un des différents éléments de suspension d'objet peut être raccordé de manière libérable.

12. Dispositif (600) selon la revendication 11, ledit mécanisme de suspension d'objet (601) étant conçu pour réaliser un essai de pression sur un objet.
